(19) Europäisches Patentamt
European Patent Office
Office européen des brevets

(11) Numéro de publication: **0 242 275 B1**

(12) **FASCICULE DE BREVET EUROPEEN**

(45) Date de publication du fascicule du brevet: **20.06.90**

(51) Int. Cl.⁵: **C08L 5/12**, C08L 33/26

(21) Numéro de dépôt: **87400785.9**

(22) Date de dépôt: **08.04.87**

(54) **Matière sèche hydratable en un gel aqueux contenant des particules de polymère dispersées, procédé pour sa préparation et son application en biologie.**

(30) Priorité: **15.04.86 FR 8605347**

(43) Date de publication de la demande: **21.10.87 Bulletin 87/43**

(45) Mention de la délivrance du brevet: **20.06.90 Bulletin 90/25**

(84) Etats contractants désignés: **AT BE CH DE ES FR GB GR IT LI LU NL SE**

(56) Documents cités:
**EP-A- 0 079 837**
**FR-A- 2 297 879**
**FR-A- 2 334 106**

(73) Titulaire: **RHONE-POULENC CHIMIE, 25, quai Paul Doumer, F-92408 Courbevoie Cédex(FR)**

(72) Inventeur: **Charmot, Dominique, 18-20, rue Mathis, F-75019 Paris(FR)**
Inventeur: **Daniel, Jean-Claude, 13, rue de Neuilly, F-94120 Fontenay/Sous/Bois(FR)**

(74) Mandataire: **Vignally, Noel et al, Rhône-Poulenc Chimie Service Brevets Chimie Centre de Recherches de Saint-Fons B.P. 62, F-69190 Saint-Fons Cédex(FR)**

**EP 0 242 275 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet une matière sèche hydratable en un gel aqueux renfermant des particules de polymère dispersées, son procédé de préparation et son application en biologie (tests de diagnostic, culture de cellules, chromatographie d'affinité ...).

La demande européenne n° 87.786 décrit des billes comprenant une matrice en un gel hydraté d'agarose renfermant des microsphères ou de la poudre d'un polyaldéhyde, de polyacroleïne par exemple ; ces billes peuvent être utilisées dans diverses applications telles que chromatographie d'affinité, hémoperfusion, résines échangeuses d'ions, tests de diagnostic ...

Les inconvénients de tels produits résident dans les problèmes rencontrés lors de leur mise en oeuvre (difficulté de synthèse des polyacroléïnes et toxicité de l'acroleïne monomère) ; de plus, ces billes se trouvent à l'état de gel humide difficilement manipulable.

Le brevet français publié sous le n° 2.297.879, décrit la préparation de plaques séchées réhydratables contenant de l'agarose ou de la gélose, utilisables dans des techniques comme l'immunodiffusion, l'électrophorèse ... ; le procédé décrit consiste à former sur un support un film de gel aqueux d'agarose ou de gélose contenant un polymère d'acrylamide hydrosoluble puis à sécher le film de gel.

Ces plaques présentent l'avantage de pouvoir être conservées un temps prolongé et de pouvoir être réhydratées au moment désiré ; par contre, elles ne peuvent être utilisables que dans un domaine restreint d'application.

Il est également connu de fixer des protéines sur des particules de polymère en dispersion aqueuse, par absorption lorsqu'il s'agit de particules de polystyrène par exemple, ou par covalence lorsqu'il s'agit de polymères présentant des groupes réactifs. L'avantage présenté par les latex résulte dans la grande surface spécifique développée par les particules et en leur large gamme de fonctions chimiques disponibles. Leur inconvénient, par contre, réside dans leur instabilité colloïdale, en particulier vis-à-vis des électrolytes, et dans la difficulté de récupération des particules sur lesquelles sont fixées les substances à isoler.

La demanderesse a trouvé une matière sèche constituée d'une matrice susceptible de former un gel poreux en présence d'eau, matrice dans laquelle sont dispersées des particules de polymère dérivé d'un monomère non miscible à l'eau ; ce produit présente l'avantage d'être aisément manipulable et d'être susceptible d'applications multiples.

La matière sèche hydratable en un gel aqueux faisant l'objet de l'invention est caractérisée en ce qu'elle comprend :

- une matrice constituée d'une substance macromoléculaire A susceptible de former un gel aqueux poreux lorsqu'elle se trouve en présence d'eau.
- un polymère linéaire hydrosoluble B
- un plastifiant de ladite substance macromoléculaire A
- et, dispersées dans ladite matrice, des particules à base d'un polymère C dérivé d'au moins un monomère non miscible à l'eau, présentant une température de transition vitreuse supérieure à 30°C (de préférence supérieure à 60°C) et possédant éventuellement des groupes fonctionnels réactifs.

La matière séche hydratable faisant l'objet de l'invention peut se présenter sous une forme quelconque : films, plaques, bâtons (sticks), pastilles, billes ...

La substance macromoléculaire A constituant la matrice peut être choisie parmi les polysaccharides, les protéines, ... dont les solutions aqueuses sont susceptibles de former un gel après refroidissement à une température de 30 à 80°C.

On peut citer à titre d'exemple les polygalactoses tels que l'agarose ou la gélose, les pectines, les protéines telles que la gélatine, le collagène.

Le polymère linéaire hydrosoluble B présent dans le produit de l'invention peut être choisi parmi les polymères hydrosolubles dont la viscosité à 22°C en solution aqueuse à 5 % en poids est inférieure à 17.000 centipoises (de préférence inférieure à 6.000 centipoises).

A titre d'exemple, on peut citer les polyéthylène glycols, l'alcool polyvinylique, la poly (vinylpyrrolidone), les hydroxyalkylcelluloses, les carboxyalkylcellulose et tout particulièrement les polyacrylamides linéaires.

On entend par "polyacrylamides" aussi bien les homopolymères linéaires d'acrylamide que les copolymères linéaires d'acrylamide et d'au moins un autre comonomère du type vinylpyrrolidone, ester acrylique ou méthacrylique du type monométhacrylate d'éthylène glycol ou monoacrylate d'éthylène glycol.

La quantité de polymère linéaire hydrosoluble B présente dans la matière sèche hydratable, correspond à environ 2 à 10 fois le poids de la matrice.

Parmi les plastifiants de la substance macromoléculaire A on peut citer les polyols tels que les glycols, le glycérol, le sorbitol, les polyéthylène glycols de masse moléculaire moyenne en poids inférieure à 400.

La quantité de plastifiant correspond à environ 1 à 3 fois le poids de la matrice.

Les particules à base de polymère C dispersées dans la matrice peuvent présenter une granulométrie de l'ordre de 0,05 à 20 µm et de préférence de 0,1 à 3 µm.

Le poids de particules mis en oeuvre correspond à environ 0,1 à 20 fois (de préférence environ 1 à 10 fois) le poids de la matrice.

Ledit polymère C dérive d'au moins un monomère insaturé non miscible à l'eau.

On entend par "monomère non miscible à l'eau" les monomères présentant une solubilité dans l'eau inférieure à 5% en poids.

Parmi ceux-ci on peut citer :

- les monomères vinylaromatiques (styrène, vinyltoluène ...)
- les alkylesters d'acides $\alpha$-$\beta$ insaturés (acrylates et méthacrylates de méthyle, éthyle ...)
- les esters d'acides carboxyliques insaturés (acétate de vinyle) ...)
- le chlorure de vinyle ; le chlorure de vinylidène

- les diènes (butadiène ...)
- ceux présentant des fonctions nitriles (acrylonitrile ...)
- les siloxanes.

Une première variante de constitution du produit faisant l'objet de l'invention consiste en ce que le polymère C est un copolymère dérivé d'au moins un monomère non miscible à l'eau et d'une faible quantité ne dépassant pas 10 % en poids, par rapport au poids total de monomères (de préférence ne dépassant pas 4 % en poids) d'un ou de comonomères portant des groupes ionogènes ou réactifs tels que - $SO_3H$, - $OSO_3H$, - $\overset{+}{N}R_3$, - COOH, OH, $NH_2$, - $NR_2$,

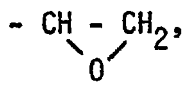

- Ø $CH_2Cl$, $CONH_2$ ...,
R représentant un radical alkyle en $C_1$ - $C_4$, de préférence en $C_1$ - $C_2$

A titre d'exemple on peut citer :
- le vinylbenzène sulfonate, les sulfoalkylesters d'acides insaturés (2-sulfoéthylméthacrylate ...).
- les acides carboxyliques insaturés (acide acrylique, méthacrylique, maleïque, itaconique ...)
- les hydroxyalkylacrylates ou méthacrylates (acrylate d'hydroxyéthyle, hydroxypropyle ...)
- les aminoalkylesters d'acides insaturés (2-aminoéthylméthacrylate ...)
- l'acrylamide
- le chlorure de vinylbenzyle
- le méthacrylate de glycidyle.

Une deuxième variante de constitution du produit faisant l'objet de l'invention consiste en ce que le polymère C est un copolymère alcali-soluble ou acido-soluble, c'est-à-dire susceptible de se solubiliser sous forme de solution aqueuse de copolymère lorsque le pH est rendu alcalin ou acide.

Ces copolymères dérivent d'au moins un monomère non miscible à l'eau et de 4 à 90 % en poids par rapport au poids total de monomères (de préférence de 10 à 50 %) d'au moins un comonomère aniogène dans le cas des copolymères alcali-solubles ou catiogènes dans le cas des copolymères acido-solubles. Le taux de comonomère est bien entendu fonction de l'hydrophylie de celui-ci.

A titre d'exemple de comonomère aniogène on peut citer les acides carboxyliques insaturés tels que les acides itaconique, maleïque, fumarique ...

Le pH de solubilisation des copolymères alcali-solubles ainsi formés est généralement de l'ordre de 6 à 11.

A titre d'exemple de comonomère catiogène on peut citer:

1) Les N(ωdialkylaminoalkyl)amides d'acides carboxyliques insaturés de formule:

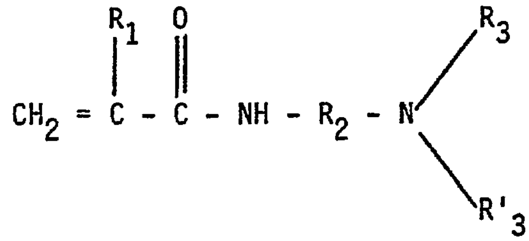

où : . $R_1$ représente un atome d'hydrogène ou un groupe alkyle en $C_1$-$C_4$, et de préférence en $C_1$-$C_2$.
$R_2$ représente un groupe alkylène $C_1$-$C_{12}$, et de préférence $C_1$-$C_8$.
. $R_3$ et $R'_3$ représentent des groupes alkyles en $C_1$-$C_6$, et de préférence en $C_1$-$C_4$, phényles éventuellement substitués par un radical alkyle en $C_1$-$C_9$.
Répondent à cette formule le diméthylaminométhyl-acrylamide ou méthacrylamide, le diméthylamino-éthyl-acrylamide ou -méthacrylamide ...

2) Les aminoesters insaturés de formule :

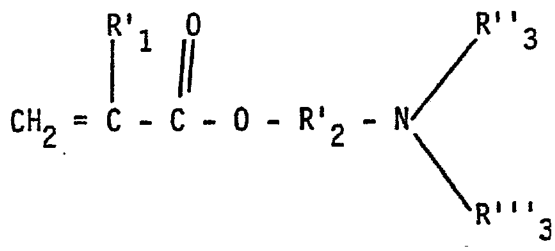

où :
. $R'_1$ représente un groupe alkyle en $C_1$-$C_5$ et de préférence en $C_1$-$C_2$,
. $R'_2$ représente un groupe alkylène linéaire ou ramifié contenant au moins 2 atomes de carbone et de préférence en $C_2$-$C_{12}$, en particulier en $C_2$-$C_8$,
. $R''_3$ et $R'''_3$ sont identiques ou différents et représentent un groupe alkyle en $C_1$-$C_6$, et de préférence $C_1$-$C_4$, éventuellement substitué par un radical hydroxy, un groupe phényle éventuellement substitué par un radical alkyle en $C_1$-$C_9$,
le nombre total d'atomes de carbone contenus dans les radicaux $R'_2$, $R''_3$ et $R'''_3$ devant être supérieur à 8 de préférence supérieur ou égal à 10.

Répondent à cette formule le diterbutylaminoéthylméthacrylate, le diterbutylaminopropylméthacrylate, le dipentylaminoéthylméthacrylate.

Le pH de solubilisation des copolymères acido-solubles ainsi formés est inférieur à 8 et de préférence inférieur à 5.

Un cas particulier de cette deuxième variante de constitution consiste en ce que le polymère C constitué d'un copolymère alcali-soluble présente une masse moléculaire moyenne en poids inférieure à 100.000 et de préférence inférieure à 50.000.

Une troisième variante de constitution du produit faisant l'objet de l'invention consiste en ce que le polymère C est "sensibilisé", ce qui signifie que des substances actives telles que anticorps, antigènes, médicaments, enzymes ... sont immobilisées sur les particules de polymère C (le terme "polymère C"

correspond à la définition donnée ci-dessus et inclut les deux variantes ci-dessus développées et tout particulièrement les polymères alcali-solubles et acido-solubles).

Une autre variante de constitution de la matière séche hydratable faisant l'objet de l'invention consiste en ce que les particules de polymère C incluses dans la matrice sont des particules de polymère magnétisables.

Lesdites particules magnétisables contiennent de 0,5 à 50% en poids (de préférence de 0,5 à 35% et tout particulièrement de 0,7 à 20%) d'une charge magnétique dont la taille est inférieure à 1 µm et de préférence comprise entre 0,002-0,05 µm ; la charge magnétique est bien évidemment suffisamment fine pour pouvoir être incluse dans les particules de polymère.

Cette charge magnétique peut être constituée par exemple par :

. des métaux ou leurs alliages tels que: fer, fer-silicium, nickel, cobalt, ou leurs alliages avec du molybdène, du chrome, du cuivre, du vanadium, du manganèse, de l'aluminium, du titane ;

. des oxydes de fer: $Fe_3O_4$ ou $\gamma$-$Fe_2O_3$ pur ou en combinaison ou en mélange avec d'autres oxydes comme les oxydes de cobalt, manganèse, zinc, baryum, terres rares;

. du dioxyde de chrome.

La matière séche hydratable faisant l'objet de l'invention peut être obtenue en réalisant les étapes suivantes :

1) mélange :

. d'une solution aqueuse d'une substance macromoléculaire A susceptible de former après refroidissement à une température de 30 à 80°C un gel aqueux poreux lorsque ladite substance se trouve en présence d'eau, à une concentration correspondant à la formation dudit gel à une température de 30 à 80°C

. d'un polymère linéaire hydrosoluble B

. d'un plastifiant de ladite substance macromoléculaire A . et d'un latex d'un polymère C dérivé d'au moins un monomère non miscible à l'eau, présentant une température de transition vitreuse supérieure à 30°C (de préférence supérieure à 60°C) et possédant éventuellement des groupes fonctionnels réactifs.

2) refroidissement jusqu'à une température inférieure à celle de gélification de la solution aqueuse de substance moléculaire A et mise en forme au cours du refroidissement du gel aqueux obtenu.

3) puis séchage à une température inférieure à ladite température de gélification.

Les matières premières mises en oeuvre pour réaliser l'étape de mélange sont celles déjà décrites ci-dessus.

Cette étape de mélange est réalisée à une température supérieure à celle de formation d'un gel aqueux de substance macromoléculaire A ; la concentration en substance macromoléculaire dans la solution aqueuse correspond à celle nécessaire à la formation d'un tel gel. Ainsi lorsque la substance macromoléculaire est de l'agarose, l'étape de mélange est réalisée à l'aide d'une solution aqueuse à 0,5 à 2 % en poids d'agarose, à une température supérieure à 40°C et généralement inférieure ou égale à 90°C.

La quantité de polymère linéaire B hydrosoluble mise en oeuvre correspond à environ 2 à 10 fois le poids de substance macromoléculaire A (sèche).

La quantité de plastifiant mise en oeuvre correspond à environ 1 à 3 fois le poids de substance macromoléculaire A (sèche).

Le polymère linéaire hydrosoluble B et le plastifiant peuvent être mis en oeuvre à l'état dissous soit en totalité dans la solution aqueuse de substance macromoléculaire A, soit en partie dans cette solution aqueuse et en partie dans le latex de polymère C.

Le latex mis en oeuvre contient de 5 à 30% (de préférence de 10 à 15%) en poids de particules de polymère.

La quantité de latex mis en oeuvre équivaut à une quantité de polymère C correspondant à 0,1 à 20 fois (de préférence environ 1 à 10 fois) le poids de substance macromoléculaire A (sèche).

Lorsque, selon la troisième variante de constitution du produit faisant l'objet de l'invention, des particules portent des substances actives immobilisées, lesdites substances actives peuvent être introduites dans le milieu, soit directement en mettant en oeuvre un latex de particules de polymère C sur lesquelles lesdites substances ont été fixées par absorption physique ou chimiquement par covalence, soit indirectement après encapsulation des particules de polymère C par le gel aqueux de substance macromoléculaire A, par migration desdites substances actives au travers du gel de substance macromoléculaire A puis immobilisation par absorption physique ou par covalence.

La fixation des molécules biologiques par covalence sur les particules de polymères peut être réalisée par réaction de couplage, réaction faisant intervenir les groupements superficiels des particules de polymère et les groupements fonctionnels de la molécule biologique à fixer.

Cette réaction de couplage peut être réalisée selon des méthodes bien connues, par exemple :

. en faisant appel à des agents de couplages (tels que glutaraldéhyle, carbodiimide hydrosoluble)

. par activation des fonctions du polymère (par exemple par diazotation, par action du bromure de cyanogène, de l'hydrazine..) puis réaction avec la molécule à fixer.

Lorsqu'une matière sèche hydratable et magnétisable est désirée, le latex de polymère mis en oeuvre est un latex dont les particules de polymère sont magnétisables.

Lesdits latex peuvent être obtenus selon des procédés connus, par exemple selon les procédés décrits dans le brevet européen N° 38.730 ou le brevet américain N° 4.157.323.

L'opération de mise en forme du gel aqueux obtenu par refroidissement est réalisée selon une méthode fonction de la forme désirée pour la matière séche finale.

Lorsque la substance macromoléculaire mise en oeuvre est de l'agarose, cette étape de refroidissement est favorablement réalisée à une température de 15 à 25°C.

Le gel aqueux, une fois mis en forme est séché sous un courant d'air, à une température voisine de celle de l'étape de refroidissement.

Un gel sous forme de film peut être obtenu en coulant le mélange obtenu à la première étape sur une plaque de verre, en laissant refroidir pour transformer le film liquide déposé en un film de gel aqueux ; le film de gel aqueux peut ensuite être démoulé et séché comme ci-dessus indiqué.

Un mode particulièrement intéressant de mise en forme du gel aqueux et de séchage est celui décrit dans le brevet français publié sous le n° 2.297.879.

Le procédé de mise en forme consiste à couler le mélange obtenu à la première étape sur un support constitué d'une plaque thermo-plastique transparente GEL-BOND commercialisée par L K B posée sur une plaque de verre horizontale, à laisser refroidir pour former un gel aqueux qui adhère à la plaque, à recouvrir le film de gel aqueux d'une feuille à base de cellulose régénérée ("Cellophane"® commercialisée par Rhône-Poulenc) imbibée d'une solution aqueuse de glycérol ; la feuille de cellulose régénérée est repliée sous la plaque de verre.

L'ensemble est ensuite séché à température ambiante sous un courant d'air ; la plaque de verre est enfin détachée de la plaque plastique revêtue de matière sèche hydratable.

La matière sèche hydratable en un gel aqueux contenant des particules de polymère dispersées faisant l'objet de l'invention, présente l'avantage d'associer les propriétés positives de la matrice, à savoir être hydratable au moment désiré en un gel aqueux poreux sous forme de films, plaques, billes ... aisément manipulables, être compatible avec des milieux aqueux même très riches en électrolytes, être perméable aux protéines de haut poids moléculaire, aux propriétés positives des particules de polymère dérivé d'un monomère non miscible à l'eau, à savoir, de posséder une surface spécifique élevée et contrôlée ainsi qu'une large gamme de fonctions chimiques disponibles en surface.

Ledit produit est particulièrement intéressant pour ses applications en biologie ; la nature poreuse du gel de substance macromoléculaire A permet aux protéines d'accéder jusqu'aux particules de polymère C et de s'y fixer par absorption ou covalence. De nombreuses substances actives telles que anticorps, antigènes, médicaments, enzymes ... peuvent ainsi être immobilisées ; selon la nature de ladite substance active immobilisée on peut obtenir un support susceptible d'être avantageusement utilisé dans les tests immunoenzymatiques ou immunoradiologiques, en chromatographie d'affinité, dans les systèmes d'épuration extra corporelle, comme catalyseur enzymatique en biotechnologie, comme dispositif contrôlant l'administration de médicament (drug release system), comme support de culture cellulaire.

Les produits de l'invention dont les particules de polymère C sont alcali-solubles ou acido-solubles avec une faible masse moléculaire (inférieure à 100.000) et sur lesquelles sont immobilisées des substances actives, sont particulièrement intéressantes en chromatographie d'affinité ; par exemple si un tel produit renfermant des particules de polymère alcali-soluble sur lesquelles ont été immobilisés des anticorps est mis en présence d'un mélange d'antigènes, l'antigène reconnu par l'anticorps se fixe sur la particule et y reste fixé après élution du mélange d'antigènes ; une simple élévation de pH permet aux particules de polymère de se dissoudre et de migrer à travers le gel poreux en libérant le complexe antigène-anticorps formé.

La propriété d'alcali-solubilité (ou d'acido-solubilité) du polymère C de masse moléculaire quelconque peut être mise à profit pour la constitution de vecteurs de médicaments où le principe actif est libéré par simple modification de pH.

Un autre avantage de la matière sèche hydratable en un gel aqueux contenant des particules de polymères dispersées faisant l'objet de l'invention, consiste en ce qu'un choix judicieux de la nature de la substance macromoléculaire A donne la possibilité de récupérer sélectivement les particules de polymère C sur lesquelles ont été fixées les substances à isoler, et ce, en détruisant après emploi la substance macromoléculaire A, par exemple par dégradation enzymatique lorsque la substance macromoléculaire A est du collagène.

Les exemples suivants sont donnés à titre indicatif et ne peuvent être considérés comme une limite du domaine et de l'esprit de l'invention.

Exemple 1

Préparation d'une plaque sèche réhydratable contenant des particules de polystyrène de diamètre 0,8 micromètre.

On dissout à température ambiante 8 g de polyacrylamide linéaire, dont la viscosité en solution aqueuse à 5% à 22°C est voisine de 6000 centipoises, dans 52 g d'eau contenant 2 g de glycérol ; cette opération dure environ 48 heures.

On ajoute ensuite à cette préparation 48 g de latex ESTAPOR® K 080 (latex commercialisé par Rhône-Poulenc et constitué de particules monodispersées de polystyrène de 0,8 micron) à 41 % d'extrait sec.

Par ailleurs on dissout au bain-marie bouillant 2 g d'agarose dans 100 ml d'eau déminéralisée contenant 2 g de glycérol.

Les deux solutions sont portées à 50°C puis mélangées en agitant lentement de façon à éviter la formation de bulles d'air.

Le mélange obtenu est coulé sur un support plastique transparent GEL-BOND (commercialisé par LKB) posé sur une plaque de verre horizontale, de façon à obtenir une couche de gel de 0,8 mm d'épaisseur.

L'ensemble est refroidi à température ambiante ; le gel aqueux adhère au support.

On recouvre ensuite le film de gel aqueux d'une feuille de "Cellophane"® (commercialisée par Rhône-Poulenc) préalablement trempée dans une solution glycéro-aqueuse à 2% de glycérol. On replie les bords de la feuille de "Cellophane"® sous la plaque de verre et on sèche l'ensemble à température ambiante sous un courant d'air durant 15 heures.

L'ensemble constitué par le film de gel séché d'aspect blanc, le support GEL-BOND et la feuille de "Cellophane"® est ensuite détaché de la plaque de verre et conservé à température ambiante.

La réhydratation du film de gel séché peut être effectuée en plongeant le film de gel séché débarrassé du support GEL-BOND et de la feuille de "Cellophane"®, dans de l'eau à température ambiante ; cette eau est renouvelée pour éliminer le polyacrylamide linéaire ; la réhydratation s'accompagne d'un gonflement important du gel sans relargage des particules de polystyrène.

La surface spécifique de la matière sèche calculée par gramme de matière sèche est de 4,4 $m^2$.

Exemple 2

Préparation d'une plaque sèche réhydratable contenant des particules de polystyrène carboxylé de diamètre 0,3 micromètre.

Les opérations décrites à l'exemple 1 sont réalisées à partir des matières premières suivantes :
. Agarose 3 g
. glycérol 6 g (3g + 3g)
. polyacrylamide 6 g
. Latex ESTAPOR® K1-030 40 g
(le latex ESTAPOR K1-030, commercialisé par Rhône-Poulenc, est une dispersion aqueuse de particules calibrées de polystyrène carboxylé ; le diamètre mesuré des particules est de 0,326 ± 0,010 micron ; le taux de fonctions COOH superficielles est de 273 microéquivalents par gramme de particules sèches ; le taux d'extrait sec est de 30%).

On obtient ainsi un gel sec réhydratable.

La surface spécifique de la matière sèche calculée par gramme de matière sèche est de 13,2 $m^2$.

Le taux de fonctions carboxyliques disponible par gramme de matrice sèche est de 192 microéquivalents.

Exemple 3

Préparation d'une plaque sèche réhydratable contenant des particules de polystyrène chlorobenzylé de diamètre 0,2 micron.

Les opérations décrites à l'exemple 1 sont réalisées à partir des matières premières suivantes :
. Agarose 1 g
. glycérol 2 g (1g + 1g)
. Alcool polyvinylique 5 g (RHODOVIOL 4-125 commercialisé par Rhône-Poulenc)
. Latex ESTAPOR K10-020 50 g
(le latex ESTAPOR K10-020, commercialisé par Rhône-Poulenc, est une dispersion aqueuse de particules calibrées de polystyrène chlorobenzylé ; le diamètre mesuré des particules est de 0,210 ± 0,006 micron ; le taux de fonctions - Ø - $Ch_2Cl$ est de 200 microéquivalents par gramme de particules sèches ; le taux d'extrait sec est de 10%).

On obtient ainsi un gel sec réhydratable.

La surface spécifique de la matière sèche calculée par gramme de matière sèche est de 11 $m^2$.

Le taux de fonctions - Ø - $CH_2Cl$ disponible par gramme de matrice sèche est de 77 microéquivalents.

La présentation du gel sous sa forme sèche permet de conserver les groupements chlorobenzyliques intacts et d'éviter leur hydrolyse. Cela représente un avantage important car il a été constaté que les latex de polystyrène chlorobenzylé présentent des problèmes de stabilité au stockage ; en effet en cours de stockage, le pH de tels latex qui est de 7-8 au départ peut descendre jusqu'à 2 avec libération d'ions chlorure et formation de groupements alcool benzylique par hydrolyse partielle des groupements chlorobenzylés présents en surface.

Exemple 4

Préparation d'un support sec réhydratable contenant des particules de polystyrène carboxylé magnétiques.

Les opérations décrites à l'exemple 1 sont réalisées à partir des matières premières suivantes :
. Agarose 2 g
. sorbitol 5 g (2,5g + 2,5 g)
. Polyvinylpyrrolidone 4 g (LUVISKOL K15 commercialisé par B.A.S.F.)
. Latex ESTAPOR MS1-070/25 150 g
(le latex ESTAPOR MS1-070.25, commercialisé par Rhône-Poulenc est une dispersion aqueuse de particules magnétiques à base de polystyrène carboxylé renfermant 25% en poids de magnétite $Fe_3O_4$ le diamètre moyen est de 0,7 micromètre; le taux de fonctions carboxyliques est de 40 microéquivalents par gramme de particules sèches; le taux d'extrait sec est de 10%).

La plaque sèche obtenue est découpée sous forme de carrés de 1 mm de côté.

On constate qu'après réhydratation les morceaux sont magnétisables à l'aide d'un aimant de laboratoire.

Exemple 5

Préparation d'une plaque sèche réhydratable contenant des particules de polymère à caractère alcali-soluble.

Les opérations décrites à l'exemple 1 sont réalisées à partir des matières premières suivantes :
. Agarose 4 g
. éthylèneglycol 4 g (2 g + 2 g)
. polyacrylamide 8 g
. latex alcali-soluble 20 g
(le latex alcali-soluble est une dispersion aqueuse de particules d'un terpolymère styrène/acide méthacrylique/acrylate d'éthyle de composition pondérale 18/41/11; la taille des particules est de l'ordre de 0,15 micromètre; le taux d'extrait sec est de 38,5%; ce terpolymère se dissout dans l'eau dès lors que le pH est supérieur à 8; sa viscosité en solution aqueuse à 10 % est inférieure à 150 centipoises à pH 9).

La plaque sèche obtenue est réhydratée par trempage dans de l'eau légèrement alcaline (pH 9);

elle devient translucide tout en gardant sa cohésion et libère dans l'eau le polymère constituant des particules.

Exemple 6

Préparation d'une plaque sèche réhydratable contenant des particules de polymère à caractère acido-soluble.

Les opérations décrites à l'exemple 1 sont réalisées à partir des matières premières suivantes :
. Agarose 4 g
. éthylèneglycol 4 g (2 g + 2 g)
. polyacrylamide 8 g
. latex acido-soluble 20 g
(le latex acido-soluble est une dispersion aqueuse de particules d'un copolymère acétate de vinyle/acrylate de diéthylaminoéthyle de composition pondérale 90/10; la taille des particules est de l'ordre de 0,15 micromètre; le taux d'extrait sec est de 38%; ce copolymère se dissout dans l'eau dès lors que le pH est inférieur à 3 ; sa viscosité en solution aqueuse à 10% est inférieure à 150 centipoises à pH 2).

La plaque sèche obtenue est réhydratée par trempage dans de l'eau neutre; la plaque réhydratée devient translucide tout en gardant sa cohésion et libère dans l'eau le polymère constituant les particules, si l'on abaisse le pH en dessous de 2 par de l'acide chlorhydrique dilué.

**Revendications**

1) Matière sèche hydratable en un gel aqueux comprenant:
- une matrice constituée d'une substance macromoléculaire A susceptible de former un gel aqueux poreux lorsqu'elle se trouve en présence d'eau ;
- un polymère linéaire hydrosoluble B
- un plastifiant de ladite substance macromoléculaire A
caractérisée en ce qe la matière comprend en outre, dispersées dans ladite matrice, des particules à base d'un polymère C dérivé d'au moins un monomère non miscible à l'eau, présentant une température de transition vitreuse supérieure à 30°C et possèdant éventuellement des groupes fonctionnels réactifs.

2) Matière sèche selon la revendication 1 caractérisée en ce que la substance macromoléculaire A est de l'agarose.

3) Matière sèche selon la revendication 1 ou 2 caractérisée en ce que le polymère linéaire hydrosoluble B est un polyacrylamide linéaire.

4) Matière sèche selon l'une quelconque des revendications précédentes caractérisée en ce que la quantité de polymère linéaire hydrosoluble B présente dans la matière sèche hydratable, correspond à environ 2 à 10 fois le poids de la matrice.

5) Matière sèche selon l'une quelconque des revendications précédentes, caractérisée en ce que le plastifiant de la substance macromoléculaire A est un polyol.

6) Matière sèche selon l'une quelconque des revendications précédentes caractérisée en ce que la quantité de plastifiant correspond à environ 1 à 3 fois le poids de la matrice.

7) Matière sèche selon l'une quelconque des revendications précédentes caractérisée en ce que le polymère C présente une température de transition vitreuse supérieure à 60°C.

8) Matière sèche selon l'une quelconque des revendications précédentes, caractérisée en ce que le polymère C dérive d'un monomère vinylaromatique, d'un alkylester d'acide α-β insaturé, d'un ester d'un acide carboxylique insaturé, du chlorure de vinyle, du chlorure de vinylidène, d'un diène conjugué, d'un monomère insaturé présentant une fonction nitrile, d'un siloxane.

9) Matière sèche selon l'une quelconque des revendications précédentes, caractérisée en ce que le polymère C dérive d'un monomère non miscible à l'eau et de moins de 10 % d'au moins un comonomère portant un groupe ionogène ou réactif.

10) Matière sèche selon la revendication 9, caractérisée en ce que le comonomère est du divinylbenzène sulfonate, un sulfoalkylester d'acide insaturé, un acide carboxylique insaturé, un hydroxyalkylacrylate ou méthacrylate, un aminoalkylester d'acide insaturé, l'acrylamide, le chlorure de vinylbenzène, le méthacrylate de glycidyle.

11) Matière sèche selon l'une quelconque des revendications précédentes, caractérisée en ce que le polymère C est un polymère alcali-soluble ou acido-soluble.

12) Matière sèche selon la revendication 11 caractérisée en ce que le polymère C alcali-soluble ou acido-soluble présente une masse moléculaire moyenne en poids inférieure à 100.000 et est sensibilisé par des substances actives.

13) Matière sèche selon l'une quelconque des revendications 1 à 10 caractérisée en ce que les particules de polymère C sont magnétisables.

14) Matière sèche selon l'une quelconque des revendications précédentes caractérisée en ce que les particules de polymère C présentent une granulométrie de l'ordre de 0,05 à 20 µm.

15) Matière sèche selon l'une quelconque des revendications précédentes caractérisée en ce que le poids des particules de polymère C correspond à environ 0,1 à 20 fois le poids de la matrice.

16) Procédé de préparation de la matière sèche hydratable faisant l'objet de la revendication 1 caractérisé en ce qu'il comprend les étapes suivantes:
1. mélange:
. d'une solution aqueuse d'une substance macromoléculaire A susceptible de former après refroidissement à une température de 30 à 80°C un gel aqueux poreux lorsque ladite substance se trouve en présence d'eau, à une concentration correspondant à la formation dudit gel à une température de 30 à 80°C
. d'un polymère linéaire hydrosoluble B
. d'un plastifiant de ladite substance macromoléculaire
. et d'un latex d'un polymère C dérivé d'au moins un monomère non miscible à l'eau, présentant une température de transition vitreuse supérieure à 30°C, et possédant éventuellement des groupes fonctionnels réactifs.

2. refroidissement jusqu'à une température inférieure à celle de gélification de la solution aqueuse de substance moléculaire A et mise en forme au cours du refroidissement du gel aqueux obtenu.

3. puis séchage à une température inférieure à ladite température de gélification.

17) Procédé selon la revendication 16, caractérisé en ce que la substance macromoléculaire A est de l'agarose.

18) Procédé selon la revendication 16 ou 17, caractérisé en ce que le polymère linéaire hydrosoluble B est un polyacrylamide linéaire.

19) Procédé selon l'une quelconque des revendications 16 à 18, caractérisé en ce que la quantité de polymère linéaire hydrosoluble mise en oeuvre correspond à environ 2 à 10 fois le poids de la matrice.

20) Procédé selon l'une quelconque des revendications 16 à 19, caractérisé en ce que le plastifiant de la substance macromoléculaire A est un polyol.

21) Procédé selon l'une quelconque des revendications 16 à 20, caractérisé en ce que la quantité de plastifiant correspond à 1 à 3 fois le poids de la matrice.

22) Procédé selon l'une quelconque des revendications 16 à 21 caractérisé en ce que le polymère C constituant le latex présente une température de transition vitreuse supérieure à 60°C.

23) Procédé selon l'une quelconque des revendications 16 à 22, caractérisé en ce que le polymère C constituant le latex dérive d'un monomère vinylaromatique, d'un alkylester d'acide $\alpha$-$\beta$ insaturé, d'un ester d'un acide carboxylique insaturé, du chlorure de vinyle, du chlorure de vinylidène, d'un diène conjugué, d'un monomère insaturé présentant une fonction nitrile, d'un siloxane.

24) Procédé selon l'une quelconque des revendications 16 à 23, caractérisé en ce que le polymère C constituant le latex dérive d'un monomère non miscible à l'eau et de moins de 10 % d'au moins un comonomère portant un groupe ionogène ou réactif.

25) Procédé selon la revendication 24 caractérisé en ce que le comonomère est le divinylbenzène sulfonate, un sulfoalkylester d'acide insaturé, un acide carboxylique insaturé, un hydroxyalkylacrylate ou méthacrylate, un aminoalkylester d'acide insaturé, l'acrylamide, le chlorure de vinylbenzyle, le méthacrylate de glycidyle.

26) Procédé selon l'une quelconque des revendications 16 à 25 caractérisé en ce que le latex de polymère C est un latex de polymère alcali-soluble ou acido-soluble.

27) Procédé selon la revendication 26 caractérisé en ce que le polymère C alcali-soluble ou acido-soluble constituant le latex présente une masse moléculaire moyenne en poids inférieure à 100.000 et est sensibilisé par des substances actives.

28) Procédé selon l'une quelconque des reendications 16 à 25 caractérisé en ce que les particules de polymère C constituant le latex sont magnétisables.

29) Procédé selon l'une quelconque des revendications 26 à 28 caractérisé en ce que les particules de polymère C constituant le latex présentent une granulométrie de l'ordre de 0,05 à 20 µm.

30) Procédé selon l'une quelconque des revendications 16 à 29 caractérisé en ce que le latex de polymère C contient de 5 à 30 % en poids de particules de polymère C.

31) Procédé selon l'une quelconque des revendications 16 à 30 caractérisé en ce que le poids des particules de polymère C correspond à environ 0,1 à 20 fois le poids de la matrice.

32) Matière sèche hydratable, sous forme de films, plaques, batons, pastilles, billes obtenue selon le procédé faisant l'objet de l'une quelconque des revendications 16 à 31.

33) Utilisation, après hydration, de la matière sèche hydratable faisant l'objet de l'une quelconque des revendications 1 à 15 et 32 en biologie.

34) Utilisation selon la revendication 33 pour l'immobilisation de substances actives.

35) Utilisation, après hydratation, de la matière sèche faisant l'objet de la revendication 11 pour la constitution de vecteurs de médicaments.

36) Utilisation, après hydratation, de la matière sèche faisant l'objet de la revendication 12 en chromatographie d'affinité.

## Claims

1. Dry material which can be hydrated into an aqueous gel comprising:
a matrix consisting of a macromolecular substance A capable of forming a porous aqueous gel when it is in the presence of water;
a water-soluble linear polymer B;
a plasticizer for the said macromolecular substance A;
characterized in that the material further comprises, dispersed in the said matrix, particles based on a polymer C derived from at least one water-immiscible monomer, which has a glass transition temperature higher than 30°C and which optionally contains reactive functional groups.

2. Dry material according to claim 1, characterized in that the macromolecular substance A is agarose.

3. Dry material according to claim 1 or 2 , characterized in that the water-soluble linear polymer B is a linear polyacrylamide.

4. Dry material according to any one of the preceding claims, characterized in that the quantity of water-soluble linear polymer B present in the dry material which can be hydrated corresponds to approximately 2 to 10 times the weight of the matrix.

5. Dry material according to any one of the preceding claims, characterized in that the plasticizer for the macromolecular substance A is a polyol.

6. Dry material according to any one of the preceding claims, characterized in that the quantity of plasticizer corresponds approximately 1 to 3 times the weight of the matrix.

7. Dry material according to any one of the preceding claims, characterized in that the polymer C has a glass transition temperature higher than 60°C.

8. Dry material according to any one of the preceding claims, characterized in that the polymer C is derived from a vinylaromatic monomer, an alkyl ester of an $\alpha$, $\beta$-unsaturated acid, an ester of an unsaturated carboxylic acid, vinyl chloride, vinylidene chloride, a conjugated diene, an unsaturated monomer which contains a nitrile group, or a siloxane.

9. Dry material according to any one of the preceding claims, characterized in that the polymer C is derived from a water-immiscible monomer and from less than 10% of at least one comonomer which carries an ionogenic or reactive group.

10. Dry material according to claim 9, characterized in that the comonomer is divinylbenzene sulphonate, a sulphoalkyl ester of an unsaturated acid, an unsaturated carboxylic acid, a hydroxyalkyl acrylate or methacrylate , an aminoalkyl ester of an unsaturated acid, acrylamide, vinylbenzene chloride or glycidyl methacrylate.

11. Dry material according to any one of the preceding claims, characterized in that the polymer C is an alkali-soluble or acid-soluble polymer.

12. Dry material according to claim 11, characterized in that the alkali-soluble or acid-soluble polymer C has a weight average molecular mass less than 100 000 and is sensitized by active substances.

13. Dry material according to any one of claims 1 to 10, characterized in that the particles of polymer C can be magnetized.

14. Dry material according to any one of the preceding claims, characterized in that the particles of polymer C have a particle size of the order of 0.05 to 2 μm.

15. Dry material according to any one of the preceding claims, characterized in that the weight of the particles of polymer C corresponds to approximately 0.1 to 20 times the weight of the matrix.

16. Process for the preparation of the dry material which can be hydrated, which forms the subject of claim 1, characterized in that it comprises the following steps:

1. mixing:

an aqueous solution of a macromolecular substance A capable of forming, after cooling to a temperature of 30 to 80°C, a porous aqueous gel when the said substance is in the presence of water, at a concentration corresponding to the formation of the said gel at a temperature of 30 to 80°C,

a water-soluble linear polymer B,

plasticizer for the said macromolecular substance,

and a latex of a polymer C derived from at least one water-immiscible monomer, which has a glass transition temperature higher than 30°C, and containing reactive functional groups, if required;

2. cooling to a temperature below the gelling temperature of the aqueous solution of the molecular substance A and shaping, during the cooling, of the aqueous gel obtained; and then,

3. drying at a temperature lower than the said gelling temperature.

17. Process according to claim 16, characterized in that the macromolecular substance A is agarose.

18. Process according to claim 16 or 17, characterized in that the water-soluble linear polymer B is a linear polyacrylamide.

19. Process according to any one of claims 16 to 18, characterized in that the quantity of the water-soluble linear polymer employed corresponds to approximately 2 to 10 times the weight of the matrix.

20. Process according to any one of claims 16 to 19, characterized in that the plasticizer for the macromolecular substance A is a polyol.

21. Process according to any one of claims 16 to 20, characterized in that the quantity of plasticizer corresponds to 1 to 3 times the weight of the matrix.

22. Process according to any one of claims 16 to 21, characterized in that the polymer C forming the latex has a glass transition temperature higher than 60°C.

23. Process according to any one of claims 16 to 22, characterized in that the polymer C forming the latex is derived from a vinylaromatic monomer, an alkyl ester of an α, β-unsaturated acid, an ester of an unsaturated carboxylic acid, vinyl chloride, vinylidene chloride, a conjugated diene, an unsaturated monomer containing a nitrile group, or a siloxane.

24. Process according to any one of claims 16 to 23, characterized in that the polymer C forming the latex is derived from a water-immiscible monomer and from less than 10% of at least one comonomer which carries an ionogenic or reactive group.

25. Process according to claim 24, characterized in that the comonomer is divinylbenzene sulphonate, a sulphoalkyl ester of an unsaturated acid, an unsaturated carboxylic acid, a hydroxyalkyl acrylate or methacrylate, an aminoalkyl ester of an unsaturated acid, acrylamide, vinylbenzyl chloride or glycidyl methacrylate.

26. Process according to any one of claims 16 to 25, characterized in that the latex of polymer C is a latex of an alkali-soluble or acid-soluble polymer.

27. Process according to claim 26, characterized in that the alkali-soluble or acid-soluble polymer C forming the latex has a weight average molecular mass lower than 100 000 and is sensitized by active substances.

28. Process according to any one of claims 16 to 25, characterized in that the particles of polymer C forming the latex can be magnetized.

29. Process according to any one of claims 16 to 28, characterized in that the particles of polymer C forming the latex have a particle size of the order of 0.05 to 20 μm.

30. Process according to any one of claims 16 to 29, characterized in that the latex of polymer C contains from 5 to 30% by weight of particles of polymer C.

31. Process according to any one of claims 16 to 30, characterized in that the weight of the particles of polymer C corresponds to approximately 0.1 to 20 times the weight of the matrix.

32. Dry material which can be hydrated, in the form of films, plates, sticks, pellets or beads, obtained according to the process which forms the subject of any one of claims 16 to 31.

33. Use, after hydration, of the dry material which can be hydrated and which forms the subject of any one of claims 1 to 15 and 32, in biology.

34. Use, according to claim 33, for the immobilization of active substances.

35. Use, after hydration, of the dry material

which forms the subject of claim 11, for forming carriers for drugs.

36. Use, after hydration, of the dry material which forms the subject of claim 12, in affinity chromatography.

## Patentansprüche

1. Zu einem wäßrigen Gel hydratisierbare Trockensubstanz enthaltend:
– eine Matrix bestehend aus einer makromolekularen Substanz A, die geeignet ist, in Gegenwart von Wasser ein poröses wäßriges Gel zu bilden;
– ein wasserlösliches lineares Polymer B
– einen Weichmacher für die genannte makromolekulare Substanz A,
dadurch gekennzeichnet, daß die Trockensubstanz außerdem in dieser Matrix dispergiert Partikel auf Basis eines Polymeren C enthält, das von wenigstens einem mit Wasser nicht mischbaren Monomeren abgeleitet ist, das eine Glasübergangstemperatur oberhalb von 30°C aufweist und gegebenenfalls reaktive funktionelle Gruppen besitzt.

2. Trockensubstanz nach Anspruch 1, dadurch gekennzeichnet, daß die makromolekulare Substanz A Agarose ist.

3. Trockensubstanz nach Anspruch 1 oder 2, dadurch gekennzeichnet, daß das lineare wasserlösliche Polymer B ein lineares Polyacrylamid ist.

4. Trockensubstanz nach einem der vorhergehenden Ansprüche, dadurch gekennzeichnet, daß die Menge an linearem wasserlöslichen Polymeren B in der hydratisierbaren Trockensubstanz ungefähr dem 2- bis 10fachen des Gewichts der Matrix entspricht.

5. Trockensubstanz nach einem der vorhergehenden Ansprüche, dadurch gekennzeichnet, daß der Weichmacher für die makromolekulare Substanz A ein Polyol ist.

6. Trockensubstanz gemäß einem der vorhergehenden Ansprüche, dadurch gekennzeichnet, daß die Menge an Weichmacher ungefähr dem 1- bis 3-fachen des Gewichts der Matrix entspricht.

7. Trockensubstanz nach einem der vorhergehenden Ansprüche, dadurch gekennzeichnet, daß das Polymer C eine Glasübergangstemperatur oberhalb von 60°C aufweist.

8. Trockensubstanz nach einem der vorhergehenden Ansprüche, dadurch gekennzeichnet, daß das Polymer C sich von einem vinylaromatischen Monomeren, einem Alkylester einer α–β-ungesättigten Säure, einem Ester einer ungesättigten Carbonsäure, Vinylchlorid, Vinylidenchlorid, einem konjugierten Dien, einem ungesättigten Monomeren mit einer Nitrilfunktion oder einem Siloxan ableitet.

9. Trockensubstanz gemäß einem der vorhergehenden Ansprüche, dadurch gekennzeichnet, daß das Polymer C sich von einem mit Wasser nicht mischbaren Monomeren ableitet sowie wenigstens 10% wenigstens eines Comonomeren das eine ionogene oder reaktive Gruppe trägt.

10. Trockensubstanz gemäß Anspruch 9, dadurch gekennzeichnet, daß das Comonomere Divinylbenzolsulfonat, ein Sulfoalkylester einer ungesättigten Säure, eine ungesättigte Carbonsäure, ein Hydroxyalkylacrylat oder -methacrylat, ein Aminoalkylester einer ungesättigten Säure, Acrylamid, das Chlorid des Vinylbenzols oder das Glycidylmethacrylat ist.

11. Trockensubstanz gemäß einem der vorhergehenden Ansprüche, dadurch gekennzeichnet, daß das Polymer C ein alkalilösliches oder säurelösliches Polymer ist.

12. Trockensubstanz gemäß Anspruch 11, dadurch gekennzeichnet, daß das alkalilösliche oder säurelösliche Polymer C eine mittlere gewichtsmäßige Molekularmasse unterhalb von 100 000 aufweist und durch aktive Substanzen sensibilisiert ist.

13. Trockensubstanz gemäß einem der Ansprüche 1 bis 10, dadurch gekennzeichnet, daß die Partikel des Polymeren C magnetisierbar sind.

14. Trockensubstanz gemäß einem der vorhergehenden Ansprüche, dadurch gekennzeichnet, daß die Partikel des Polymeren C eine Granulometrie in der Größenordnung von 0,05 bis 20 µm aufweisen.

15. Trockensubstanz gemäß einem der vorhergehenden Ansprüche, dadurch gekennzeichnet, daß das Gewicht der Partikel des Polymeren C ungefähr dem 0,01- bis 20fachen des Gewichts der Matrix entspricht.

16. Verfahren zur Herstellung der hydratisierbaren Trockensubstanz gemäß Anspruch 1, dadurch gekennzeichnet, daß es die nachfolgenden Schritte umfaßt:
1. mischen:
– einer wäßrigen Lösung einer makromolekularen Substanz A, die geeignet ist, nach Abkühlung auf 30 bis 80°C in Gegenwart von Wasser ein poröses wäßriges Gel zu bilden, in einer der Bildung dieses Gels bei einer Temperatur von 30 bis 80°C entsprechenden Konzentration
– eines linearen wasserlöslichen Polymeren B
– eines Weichmachers für diese makromolekulare Substanz
– und eines Latex eines von wenigstens einem mit Wasser nicht mischbaren Monomeren abgeleiteten Polymeren C, das eine Glasübergangstemperatur oberhalb von 30°C aufweist und gegebenenfalls reaktive funktionelle Gruppen besitzt
2. Abkühlung bis auf eine Temperatur unterhalb der Gelbildungstemperatur der wäßrigen Lösung der makromolekularen Substanz A und Formen des erhaltenen wäßrigen Gels im Verlauf der Abkühlung
3. anschliessende Trocknung bei einer Temperatur unterhalb der Gelbildungstemperatur.

17. Verfahren nach Anspruch 16, dadurch gekennzeichnet, daß die makromolekulare Substanz A Agarose ist.

18. Verfahren nach Anspruch 16 oder 17, dadurch gekennzeichnet, daß das wasserlösliche lineare Polymer B ein lineares Polyacrylamid ist.

19. Verfahren gemäß einem der Ansprüche 16 bis 18, dadurch gekennzeichnet, daß die Menge des wasserlöslichen linearen Polymeren, die eingesetzt wird, ungefähr dem 2- bis 10fachen des Gewichts der Matrix entspricht.

20. Verfahren gemäß einem der Ansprüche 16 bis 19, dadurch gekennzeichnet, daß der Weichmacher für die makromolekulare Substanz A ein Polyol ist.

21. Verfahren gemäß einem der Ansprüche 16 bis 20, dadurch gekennzeichnet, daß die Menge an Weichmacher dem 1- bis 3fachen des Gewichts der Matrix entspricht.

22. Verfahren gemäß einem der Ansprüche 16 bis 21, dadurch gekennzeichnet, daß das den Latex bildende Polymer C eine Glasübergangstemperatur oberhalb von 60°C aufweist.

23. Verfahren gemäß einem der Ansprüche 16 bis 22, dadurch gekennzeichnet, daß das Polymere C, das den Latex bildet, sich von einem vinylaromatischen Monomeren, einem Alkylester einer α-β-ungesättigten Säure, einem Ester einer ungesättigten Carbonsäure, Vinylchlorid, Vinylidenchlorid, einem konjugierten Dien, einem ungesättigten Monomeren mit einer Nitrilfunktion, einem Siloxan ableitet.

24. Verfahren gemäß einem der Ansprüche 16 bis 23, dadurch gekennzeichnet, daß das Polymere C, das den Latex bildet, sich von einem mit Wasser nicht mischbaren Monomeren und wenigstens 10% wenigstens eines Comonomeren, das eine ionogene oder reaktive Gruppe trägt, ableitet.

25. Verfahren nach Anspruch 24, dadurch gekennzeichnet, daß das Comonomere das Divinylbenzolsulfonat, ein Sulfoalkylester einer ungesättigten Säure, eine ungesättigte Carbonsäure, ein Hydroxyalkylacrylat oder -methacrylat, ein Aminoalkylester einer ungesättigten Säure, Acrylamid, das Chlorid des Vinylbenzols, das Glycidylmethacrylat ist.

26. Verfahren gemäß einem der Ansprüche 16 bis 25, dadurch gekennzeichnet, daß der Latex des Polymeren C ein Latex eines alkalilöslichen oder säurelöslichen Polymeren ist

27. Verfahren gemäß Anspruch 26, dadurch gekennzeichnet, daß das den Latex bildende alkalilösliche oder säurelösliche Polymere C eine mittlere gewichtsmäßige Molekularmasse unterhalb von 100 000 aufweist und durch aktive Substanzen sensibilisiert wird.

28. Verfahren gemäß einem der Ansprüche 16 bis 25, dadurch gekennzeichnet, daß die Partikel des Polymeren C das den Latex bildet, magnetisierbar sind.

29. Verfahren gemäß einem der Ansprüche 16 bis 28, dadurch gekennzeichnet, daß die Partikel des den Latex bildenden Polymeren C eine Granulometrie in der Größenordnung von 0,05 bis 20 µm aufweisen.

30. Verfahren gemäß einem der Ansprüche 16 bis 29, dadurch gekennzeichnet, daß der Latex des Polymeren C 5 bis 30 Gewichtsprozent an Partikeln des Polymeren C enthält.

31. Verfahren gemäß einem der Ansprüche 16 bis 30, dadurch gekennzeichnet, daß das Gewicht der Partikel des Polymeren C ungefähr dem 0,1- bis 20-fachen des Gewichts der Matrix entspricht.

32. Hydratisierbare Trockensubstanz in Form von Filmen, Platten, Stäben, Pastillen, Kugeln, die nach dem Verfahren gemäß einem der Ansprüche 16 bis 31 erhalten wurden.

33. Verwendung der hydratisierbaren Trockensubstanz gemäß einem der Ansprüche 1 bis 15 und 32 nach Hydratisierung in der Biologie.

34. Verwendung gemäß Anspruch 33 zur Immobilisierung von Wirkstoffen.

35. Verwendung der Trockensubstanz gemäß Anspruch 11 nach Hydratisierung zur Herstellung von Vektoren für Medikamente.

36. Verwendung der Trockensubstanz gemäß Anspruch 12 nach Hydratisierung in der Affinitätschromatografie.